# EUROPEAN PATENT APPLICATION

(11) **EP 1 862 169 A1**
(43) Date of publication of application: **05.12.2007**
(21) Application number: 06300497.2
(22) Date of filing: 19.05.2006
(51) Int. Cl.: A61K 33/24, A61P 35/00

(54) **Use of cis-rhenium (III) diadamantate compounds for potentiating the antitumoral activity of platinum complexes**

(71) Applicant: SOCIETE DE COORDINATION DE RECHERCHES THERAPEUTIQUES, 20220 Algajola (FR)
(72) Inventor: Collery Philippe, 20200 Bastia (FR); Shtemenko, Alexander V., 49107 Dnepropetrovsk (UA); Shtemenko, Natalia I., 49107 Dnepropetrovsk (UA)
(74) Representative: Gillard, Marie-Louise

(57) **Abstract**

The present invention relates to pharmaceutical associations comprising, as active principles, a cytotoxic platinum complex and a cluster rhenium compound of formula (I) 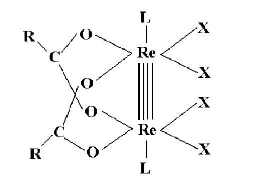 in which:
- X is chlorine or bromine;
- R is the residue of adamantanecarboxylic acid;
- L is H₂O; CH₃CN, DMPA, DMAA, DMSO or pyridine.

Application: potentiation of the antitumoral activity of cytotoxic platinum complexes.

## Description

The present invention relates to the treatment of cancers and more particularly to the use of cis-rhenium (III) diadamantate compounds for potentiating the antitumoral activity of cytotoxic platinum complexes.

The present invention also relates to novel pharmaceutical associations comprising as active principles a cytotoxic platinum complex and a cis-rhenium (III) diadamatate compound.

The two above active principles may be associated in a single pharmaceutical composition with a pharmaceutically acceptable vehicule.

The two active principles may be also conditioned separately in order to allow their sequential administration. In that case, each active principle is preferably in combination with a pharmaceutically acceptable vehicule.

The main platinum complexes known as cytotoxic agents are the cisplatin, carboplatin, oxaliplatin and nedaplatin. Regarding the antitumoral activity of these platinum complexes, the following references may be cited:
- Boisdron-Celle M, et al. Pharmacocinétique clinique comparative des dérivés du platine. Bulletin du Cancer 2001, 88 : 14-19 ;
- Koshiyama M, et al. Chemosensitivity testing of a novel platinum analog, nedaplatin (254-S), in human gynecological carcinomas: a comparison with cisplatin. Anticancer Res. 2005 Nov-Dec;25(6C):4499-502 ;
- Saint-Lorant G et al. Therapie 2005 Sept-Oct; 60(5): 499-505).

Some rhenium compounds were found to have varying degrees of effectiveness against some tumoral cells, but they are quite susceptible to decomposition in aqueous solutions and required very high doses to achieve maximum effectiveness. (Eastland G.W., et al. Studies of Rhenium Carboxylates as Antitumor Agents. Part II. Antitumor Studies of Bis(µ-Propionato) Diaquotetrabromodirhenium(III) in Tumor-Bearing Mice. Meth and Find Exptl Clin Pharmacol 1983; 5, n°7:435 -8).

It has now been found that cluster rhenium compounds with adamantane carboxylic residues as ligands do not prevent alone the tumor growth but are very useful as potentiating agents of cytotoxic platinum complexes.

The cluster rhenium compounds useful according to the invention are the compounds of formula (I): 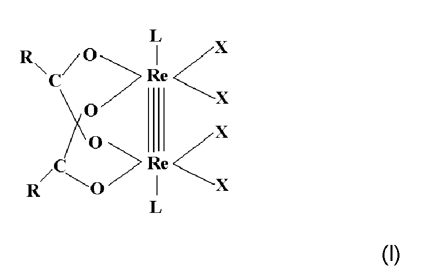 in which:
- X is chlorine or bromine;
- R is the residue of adamantanecarboxylic acid;
- L is H₂O; CH₃CN, DMPA, DMAA, DMSO or pyridine.

In the present description, the abrevations DMPA, DMAA and DMSO have the following meanings:
DMPA = dimethylphosphamide;
DMAA = dimethylacetamide;
DMSO = dimethylsulphoxide.

These compounds are described in the following papers or may be easily obtained by the processes disclosed in these papers:
- Shtemenko A. V. et al. Chemistry of binuclear rhenium clusters ; TMS publication, Pennsilvania 1997-In book "Rhenium and Rhenium Alloys" pp 189-197;
- Golichenko A.A. et al. Synthesis and Properties of Binuclear Cluster Rhenium(III) Compounds with Adamantanecarboxylic Acids. Russ. Journal of Coord. Chemistry 2006; 32, n°4: 237-46.
- Shtemenko A.V., et al. Synthesis of Novel Tetracarboxylato Dirhenium(III) Compounds and Crystal Structure of them. Z. Naturforsch 2002; 56b:381-5.

The preferred compound among the above compounds is the compound of formula:

cis [Re₂ (AdCOO)₂CL₄].2CH₃CN

in which AdCOO is the residue of adamantane-carboxylic acid.

The present invention also relates to the use of the cluster rhenium compounds of formula (I) for the preparation of medicaments useful for potentiation of the antitumoral activity of platinum complexes.

The present invention also relates to the use of the cluster rhenium compounds of formula (I) in association with cytotoxic platinum complexes for the preparation of antitumoral agents.

The amount of rhenium coumpounds of formula (I) to be used depends on the tumors to be treated and the state of the patient. Generally the dose to be used may vary between 1 and 100 mg per kg of the body of the patient to be treated.

The present invention will be described in more details by the following non limiting

### examples.

### EXAMPLE 1: Pharmacological activities

Cisplatin and cluster rhenium compound of formula:

cis-[Re₂(AdCOO)₂Cl₄].2CH₃CN, hereinafter named Re2, were tested.

Wistar rats weighting 100-120 g were inoculated by tumor carcinoma Guerink (T8) cells (Experimental Oncology, 2003, Vol. 25, pp64-68). The cells were taken in the Institute of Oncology and Experimental Pathology by R.E.Kavetskiy (Kiev). The single intraperitoneal administration of Cisplatin at the dose of 8 mg/kg was made on the 9 day after the tumor inoculation according to the method disclosed by Taylor S.K. in Medical Hypothesis 2003; 60; n°1, pp 89-93. The intraperitoneal administration of the Re2 at the dose of 7µM/kg according to the schema of antioxidant therapy [Meerson F.Z. et al. Pat. Physiol. and Exp. Therap.(Rus) 1983;N5:25-9] in liposome forms [Sktemenko et al. Ukr.Biokhim.Journal (Ukr) 2002;74,N°6:38-43] began on the 3 day after the inoculation of the tumor cells and was repeated every 2 days until day 21. Body weights were measured from day 2 after tumor inoculation, the volume of tumors were assessed from the day 7. On the day 21 animals were sacrified under chlorophormium anesthesia and the tumors were isolated and weighted. Concentrations of hemoglobine (Hb) and quantity of red blood cells (RBC) were measured according to the method described by Chevary et al. in Laboratornoe delo (Rus) 1991;N°10:9-13

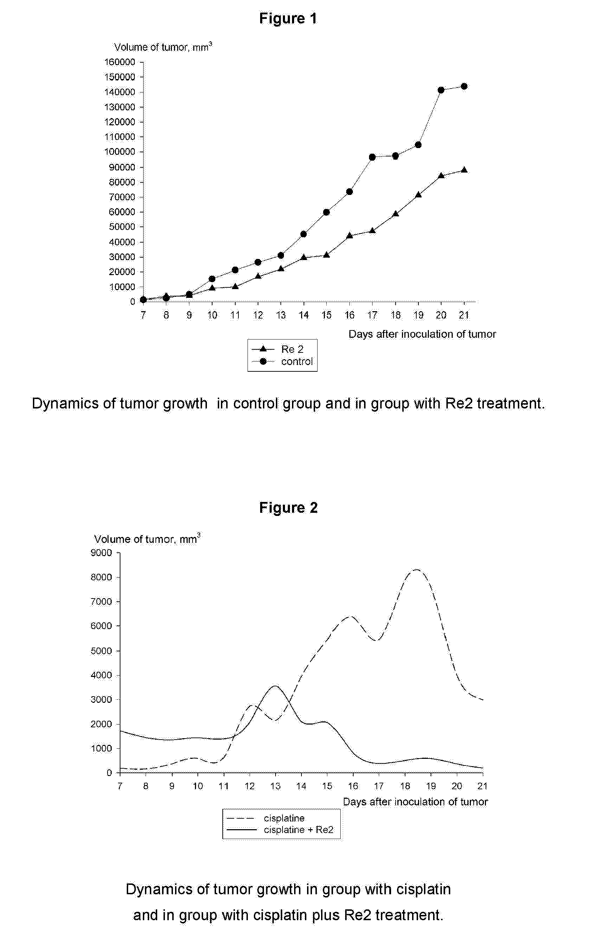

Four groups of 15 animals in each were compared: control group; group with introduction of cisplatin; group with introduction of Re2; and group with introduction of cisplatin together with Re2. Wilcoxon non parametric tests were used to compare the tumor volumes according to the absence of treatment and each group of treatment or between 2 treated groups.
Growth of the tumors in control group was very rapid and reached 146000 mm³ and occupied approximately 1/3 of the animal weight. Dynamics of tumor growth is represented on the Figure 1.

The treatments by Re2 alone did not prevent tumor growth but caused a little delay in progression of growth.
A significant decrease in measured tumor volumes was shown in cisplatin and cisplatin plus Re2 treated groups of animals (Fig. 2) in comparison to control group.

Especially little sizes of measured volumes of tumors were found on the last stages of these experiments. If influence of cisplatin and Re2 on tumor growth is compared, a great difference in the area 15-20 days of development of tumor is observed. Also, at the last day 21 the decrease was significant for rhenium group combined with cisplatin in comparison to the treatment by cisplatin alone. Taking into account practically absence of effect for Re2 alone, these results establish synergistic action of both substances.

Weight of isolated tumors and Hb levels in average were the same as in control group as in group treated by Re2 (Table 1).

**Table 1. Weight of isolated tumors (m, g) and concentration of Hb (Hb, g/l) in blood of animals on the 21 day after tumor cells inoculation**

| Group | mg | Hb, g/l |
|---|---|---|
| control | 44.87 ± 25.19 | 89.86 ± 10.36 |
| Re2 | 38.27 ± 16.77 | 72.02 ± 11.12 |
| cisplatin | 9.88 ± 9.90 | 129.49 ± 12.43 |
| cisplatin + Re2 | 3.92 ± 2.55 | 95.75 ± 9.04 |

Small isolated tumors were found in both next groups, especially under synergistic effect of cisplatin and Re2. There was a significant increase in hemoglobin in cisplatin and cisplatin with Re treated groups of animals in comparison to control group. This may be explained by the efficacy in tumor growth prevention. The rhenium compounds did not significantly modify the hemoglobin values in comparison to the administration of cisplatin alone, but there are additional experimental data, presented in Table 2, which showed that level of Hb is not the only parameter that may appreciate anemic state of an organism.

**Table 2. Morphological forms of RBC in blood of animals on the 21 day after tumor cells inoculation, %**

| Group | Discocites | Echinocites | Damaged RBC |
|---|---|---|---|
| normal | 65.00 ± 6.12 | 23.30 ± 3.12 | 11.70 ± 2.16 |
| control | 8.47 ± 1.88 | 52.57 ± 3.66 | 58.96 ± 4.54 |
| Re2 | 56.33 ± 4.32 | 22.14 ± 2.16 | 21.53 ± 2.34 |
| cisplatin | 46.36 ± 3.18 | 24.99 ± 3.84 | 28.65 ± 3.98 |
| cisplatin + Re2 | 60.67 ± 5.54 | 30.18 ± 4.08 | 9.15 ± 2.08 |

The statistical tests were carried out by the method of Wilcoxon and it was found that the p value was lower than 0.05 at day 21 between the cisplatin group and the cisplatin + Re2 group.

Development of malignancy led to morphological shift to the side of damaged forms of RBC and reversible forms - echinocites. You see, introduction of Re2 supported quantities of discocites and ehinocites on a rather high level, nevertheless did not prevent tumor growth. And in the experiment with cisplatin introduction of rhenium substance led to practically normal morphological picture of RBC, even to reduction of quantities of damaged cells.

## Claims

1. Pharmaceutical association comprising, as active principles, a cytotoxic platinum complex and a cluster rhenium compound of formula (I) 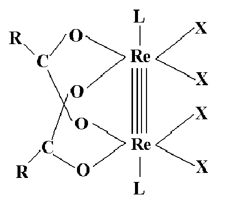 in which:
- X is chlorine or bromine;
- R is the residue of adamantanecarboxylic acid;
- L is H₂O; CH₃CN, DMPA, DMAA, DMSO or pyridine.

2. Pharmaceutical association according to claim 1, wherein the active principles are in combination with a pharmaceutically acceptable vehicule.

3. Pharmaceutical association according to claim 1, wherein the active principles are conditioned separately.

4. Pharmaceutical association according to claim 3, wherein each active principle is in combination with a pharmaceutically acceptable vehicule.

5. Use of a cluster rhenium compound of formula (I) 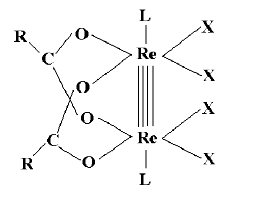 in which:
- X is chlorine or bromine;
- R is the residue of adamantanecarboxylic acid;
- L is H₂O CH₃CN, DMPA, DMAA, DMSO or pyridine.
as potentiating agent of cytotoxic platinum complexes.

6. Use of a cluster rhenium compound of formula (I) 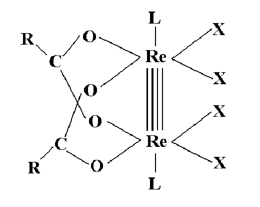 in which:
- X is chlorine or bromine;
- R is the residue of adamantanecarboxylic acid;
- L is H₂O CH₃CN, DMPA, DMAA, DMSO or pyridine
in association with a cytotoxic platinum complex for the preparation of an antitumoral agent.
